(19) Europäisches Patentamt
European Patent Office
Office européen des brevets

(11) **EP 4 678 099 A1**

(12) **EUROPEAN PATENT APPLICATION**

(43) Date of publication:
**14.01.2026 Bulletin 2026/03**

(21) Application number: **24187640.8**

(22) Date of filing: **10.07.2024**

(51) International Patent Classification (IPC):
**A61B 5/08** *(2006.01)*    **A61B 5/11** *(2006.01)*
**A61B 5/113** *(2006.01)*    **A61B 5/00** *(2006.01)*

(52) Cooperative Patent Classification (CPC):
**A61B 5/0816; A61B 5/1128; A61B 5/1135;**
**A61B 5/7207; A61B 5/7235; A61B 5/7257;**
**G08B 21/06**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **DERKX, Rene Martinus Maria**
**Eindhoven (NL)**
• **VAN DALFSEN, Age Jochem**
**Eindhoven (NL)**
• **VAN GASTEL, Mark Josephus Henricus**
**5656AG Eindhoven (NL)**

(74) Representative: **Philips Intellectual Property &**
**Standards**
**High Tech Campus 52**
**5656 AG Eindhoven (NL)**

(54) **METHOD, DEVICE AND SYSTEM FOR DETERMINING RESPIRATORY INFORMATION OF A SUBJECT**

(57)    The present invention relates to a method, device and system for determining respiratory information of a subject. To enable the determination of respiratory information in a more robust and precise way, the method comprises obtaining a chest video signal of the subject, the chest video signal including a time series of images of the subject acquired over time and covering at least part of the subject's chest area; computing a chest displacement signal from the obtained chest video signal by determining vertical displacements of the chest in a direction substantially along or parallel to the subject's longitudinal body axis; computing a chest deformation signal from the obtained chest video signal by determining deformations of the chest; computing a noise-estimate related to the non-respiratory noise contribution in the chest displacement signal using the chest displacement signal and the chest deformation signal; removing at least part of the non-respiratory noise contribution in the chest displacement signal by subtraction of the noise-estimate from the chest displacement signal to obtain a noise-reduced chest displacement signal; and determining the respiratory information from the noise-reduced chest displacement signal.

FIG.4

**Description**

FIELD OF THE INVENTION

The present invention relates to a method, device and system for determining respiratory information of a subject.

BACKGROUND OF THE INVENTION

Monitoring of vital signs, like pulse rate or respiration rate, can be done by using equipment like video, radar, accelerometers and inductive sensors (e.g., in the seat or seatbelt) and can be used to assess a person's health status or a person's stress status. This status can be used to ensure the safety of the person driving the car and also ensures the safety of passengers or other people in the vicinity of the car. Such automotive applications are upcoming because modern vehicles are now fitted with interior cameras that can sense visual modalities. Given that an interior camera is already installed in vehicles for driver monitoring purposes to prevent accidents, there is an opportunity to use them for monitoring vital signs of the driver of the car. The driver's vital signs can predict or indicate critical events, such as sudden heart attacks, strokes, or fatigue, in the early stages. This has the potential to enable a controlled stop of the vehicle before accidents are occurring.

There are many other areas of application of monitoring respiration or other respiratory information in several non-ambulant applications by using video in settings like a general ward, dialysis ward, etc. In such applications, the small breathing-induced displacement signals of the chest area are captured by video or motion tracking. Optionally, for better localization and improved quality, multiple region-of-interests (ROIs) of the chest area can be captured, wherein a statistical technique like Principal Component Analysis (PCA) may be used to combine these signals to construct the final respiration signal.

For respiration monitoring in automotive applications, there is much non-respiratory chest displacement in frequencies corresponding to typical respiration frequencies, caused by vibrations due to non-uniform road conditions. Due to the extra vertical displacements of the chest caused by vibrations, it is difficult to directly use an approach as mentioned above according to which vertical chest displacements, i.e., chest displacements in a direction along or parallel to the longitudinal direction of the subject's body, are being used directly for capturing the respiration. Depending on the type of the road and/or the type of the car, the extra displacements can be as strong or even stronger than the chest displacements caused by respiration.

An exact reference of this non-respiratory chest displacement is difficult to measure. An option would be to measure the vibrations of the seat on which the driver is sitting via camera observation or using accelerometers or inertial measurement units (IMUs). However, the (possibly time-varying) transfer function between the vibrations in the seat and the vertical displacements on the chest due to these vibrations are expected to be hard to model.

SUMMARY OF THE INVENTION

It is an object of the present invention to enable the determination of respiratory information in a more robust and precise way, in particular in automotive applications or other applications where noise is disturbing any signals used for the determination of the respiratory information.

In a first aspect of the present invention a method for determining respiratory information of a subject is presented comprising:

- obtaining a chest video signal of the subject, the chest video signal including a time series of images of the subject acquired over time and covering at least part of the subject's chest area;
- computing a chest displacement signal from the obtained chest video signal by determining vertical displacements of the chest in a direction substantially along or parallel to the subject's longitudinal body axis;
- computing a chest deformation signal from the obtained chest video signal by determining deformations of the chest;
- computing a noise-estimate related to the non-respiratory noise contribution in the chest displacement signal using the chest displacement signal and the chest deformation signal;
- removing at least part of the non-respiratory noise contribution in the chest displacement signal by subtraction of the noise-estimate from the chest displacement signal to obtain a noise-reduced chest displacement signal; and
- determining the respiratory information from the noise-reduced chest displacement signal.

In a further aspect of the present invention a system for determining respiratory information of a subject is presented comprising:

- an imaging unit configured to acquire a chest video signal of the subject, the chest video signal including a time series of images of the subject acquired over time and covering at least part of the subject's chest area; and

- a device as disclosed herein for determining respiratory information of the subject from the chest video signal.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, system, computer program and medium have similar and/or identical preferred embodiments as the claimed method, in particular as defined in the dependent claims and as disclosed herein.

The present invention is particularly focusing on the monitoring of respiratory information, for instance respiration and respiration rate of the driver, by using a video signal that may be obtained by a single 2D camera. The present invention is based on the idea to remove noise caused e.g. by vibrations of a seat on which the subject is sitting (e.g. a seat of a driver of a car, bus, lorry, train, etc.) by solely using the (video) information of the chest area of the subject. The vertical chest displacement signal is used, which may be obtained in a conventional way, which is augmented by exploiting a chest deformation signal that is capturing the deformations of the chest. The two signals (displacement signal and deformation signal) are being used to compute a noise-estimate related to the non-respiratory noise contribution in the chest displacement signal, e.g. to estimate the chest displacements caused by vibrations. This non-respiratory noise contribution in the chest displacement signal can then be removed by subtraction of the noise-estimate from the chest displacement signal. In this way, a cleaned version of the vertical displacement signal is obtained which is free of the noise, e.g. free of influences of the vibrations.

There are generally many different ways to compute the chest displacement signal and the chest deformation signal. In a preferred embodiment, the chest displacement signal is computed using an object tracker on the chest area to generate a vertical tracking signal corresponding to the displacements of the chest in the vertical direction (i.e., in the longitudinal direction of the subject's body). For instance, the chest displacement signal may be captured by tracking the full vertical displacement of the chest using a method as, e.g., described in M. Villarroel, et.al. "Non-contact vital-sign monitoring of patients undergoing haemodialysis treatment", Sci rep. 2020; vol. 10: 18529, https://doi.org/10.1038/s41598-020-75152-z. Besides the desired vertical displacement of the chest, there is also a disturbance vibration caused by e.g. the poor road conditions, which is typically also strong in the vertical direction and is superimposed on the vertical chest displacements. It is assumed in this context that the camera is in front of the subject, e.g. a driver of a car; in practical cases, the camera may be mounted in the rear-view mirror or the A-pillar of the car. In such cases, the respiratory information as well as the vibration disturbance will be also (partly) present in the x-direction of the camera.

In an embodiment, the steps of computing the chest deformation signal includes using at least two object trackers on the chest are, each object tracker tracking a different part of the chest area in two dimensions to generate at least two tracking signals in orthogonal directions; removing common-mode displacements in the at least two tracking signals; and combining the at least two tracking signals via a weighted summation. The weights may be positive values or negative values or zero.

For instance, the chest deformation signal may be captured by tracking the deformation of the chest, as, e.g., described in H. Hwang, et.al., "A Real-Time Remote Respiration Measurement Method with Improved Robustness Based on a CNN Model", Appl. Sci. 2022, 12, 11603, pp. 1-27. Another exemplary method to measure chest deformation signals is e.g. by looking at curvature-changes of edges or computing strains, as, e.g., disclosed in S. N. Grama and· S. J. Subramanian, "Computation of Full-field Strains Using Principal Component Analysis", July 2014. The deformation will generally not only be vertically oriented (like in the chest displacement signal) but will generally be present in both the x-direction (perpendicular to the vertical direction) and y-direction (corresponding to the vertical direction). The exact strengths of these components are not known a-priori, because it depends on other factors, like, e.g., the type of clothing, the attachment of the seatbelt, lighting conditions, etc. Besides the respiratory information (in both the x- and y-directions), this chest deformation signal will also contain noise influences, e.g. due to slight steering-wheel actions which will also result in deformations of the chest. Below a preferred embodiment to compute the chest deformation signal will be explained in detail. In another embodiment, the chest deformation signal may be constructed by a summation of differential motion signals on the chest area (both in x- and y-direction). As a result of the differential operator, the common mode displacements (as measured in the chest displacement signal) are generally not part of the chest deformation signal.

In another embodiment, the steps of computing the noise-estimate and removing the non-respiratory noise contribution are performed in the time domain and include:

- computing a calibrated chest deformation signal from the chest deformation signal and the chest displacement signal to match the phase and magnitude of the respiratory contribution in the chest deformation signal and the chest displacement signal;

- estimating the noise signal from the calibrated chest deformation signal and the chest displacement signal;
- computing a weighted noise-estimate signal, where the weight varies from 0 to 1 and the weight is representing the similarity of the respiratory contribution in the calibrated chest deformation signal and the chest displacement signal;
- removing the non-respiratory noise contribution in the chest displacement signal by subtraction of the weighted noise-estimate from the chest displacement signal.

Hereby, the computing of the calibrated chest deformation signal can be effectively performed by using adaptive filters. Furthermore, the weight for computing the weighted noise-estimate signal can be obtained by computing the similarity between the calibrated chest deformation signal and the chest displacement signal, potentially also in the frequency range where the respiratory contribution can be expected. The weight factor can be time-averaged to avoid strong discontinuities over time in the resulting output signal after removing the weighted noise-estimate.

In another embodiment, the steps of computing the noise-estimate and removing the non-respiratory noise contribution are performed in the spectral domain and include:

- computing the chest displacement spectrum from the chest displacement signal;
- computing the chest deformation spectrum from the chest deformation signal;
- estimating a noise spectrum related to the non-respiratory noise contribution in the chest displacement spectrum using the chest displacement spectrum and the chest deformation spectrum; and
- modifying the spectral energy in the chest displacement spectrum using the estimated noise spectrum.

Hereby, the phase is left unaltered to overcome the problem with the sign ambiguity between the chest displacement signal and the chest deformation signal. Modifying the spectral energy in the chest displacement spectrum includes according to an embodiment: computing the chest displacement magnitude spectrum; and subtracting the noise spectrum from the chest displacement magnitude spectrum using spectral subtraction. According to the present invention the chest displacement signal and the chest deformation signal are combined in an optimal way to obtain an improved version of the respiration that is free of the vibration influences. The fact that the chest displacement signal does not have a sign ambiguity, since the direction of the displacements are always going upward for inspirations and down for expirations, may hereby be exploited. The phase information of the chest displacement signal is kept, and the chest deformation signal is exploited to first estimate a noise (or vibration) signal of the noise in the chest displacement signal. A spectral subtraction technique may be used, as, e.g., disclosed in S.F. Boll, S,F, "Suppression of Acoustic Noise in Speech Using Spectral Subtraction", 1979, IEEE Transactions on Acoustic, Speech and Signal Processing, vol 27, pp. 113-120, to effectively subtract the noise (in the spectral domain) from the chest displacement signal. This spectral subtraction will modify (i.e. suppress) spectral energy of the original chest displacement signal but keeps the phase information of the original chest displacement signal. The main benefit of estimating a noise spectrum to use in a spectral subtraction scheme is that it allows for over-subtraction of the noise spectrum estimate. It shall be noted that the noise spectrum may particularly be a noise magnitude spectrum, but alternatively a power spectrum or a spectrum of higher order may be used as well.

Furthermore, in an embodiment the spectral subtraction uses an over-estimation of the noise spectrum with a value larger than 1. In case of an under-estimation of the noise spectrum, the spectral subtraction may not be able to fully suppress the noise from the chest displacement signal. By using an over-estimation of the noise spectrum (which may also be realized by an over-subtraction with a factor), the noise will be fully suppressed, eventually at the cost of a small attenuation of the respiratory energy in the chest displacement signal. Typical values of the over-estimation of the noise spectrum can be between 1.0 and 2.0.

In an embodiment, estimating the noise spectrum related to the non-respiratory noise contribution in the chest displacement spectrum includes computing a calibrated chest deformation spectrum from the chest deformation spectrum and the chest displacement spectrum; and estimating the noise spectrum from the calibrated chest deformation spectrum and the chest displacement spectrum. This calibration may be useful because the respiratory energy in the deformation signal may not be matching with the respiratory energy in the displacement signal. Hence, this energy may be aligned in order to properly estimate the noise spectrum (in the frequency domain).

Hereby, in an embodiment the calibrated chest deformation spectrum is computed by multiplying the chest deformation spectrum with a calibration factor; and the noise spectrum is computed by subtracting the calibrated chest deformation magnitude spectrum from the chest displacement magnitude spectrum and clipping the negative values of the noise spectrum to zero. The calibration factor may be computed by identifying the harmonic frequency that has the strongest joint energy contribution in both the chest displacement spectrum and the chest deformation spectrum; and computing the calibration factor by dividing the magnitude of the identified harmonic frequency in the chest displacement spectrum by the magnitude of the identified harmonic frequency in the chest deformation spectrum. Exemplary values of the calibration factor may be in the range of 0.1 up to 10.0. Optionally, the calibration factor may be computed by identification of the harmonic frequency only in the spectral region that is relevant for typical respiration, i.e. in the spectral region from 0.1 up to 1 Hz (corresponding to respiratory rates from 6 up to 60 breaths/min).

The method according to the present invention may further comprise selecting and/or tracking the chest area in the time series of images of the subject to generate the chest video signal. This further improves the accuracy of the determined respiratory information.

Different physiological parameters of respiratory information may be obtained by use of the present invention. Exemplary respiratory information includes one or more of respiration rate, respiration depth, respiration volume, inhalation and exhalation of the subject.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of an embodiment of a system according to the present invention.
Fig. 2 shows a schematic diagram of an embodiment of a device according to the present invention.
Fig. 3 shows a schematic diagram of a first embodiment of a method according to the present invention.
Fig. 4 shows a schematic diagram of a second embodiment of a method according to the present invention.
Fig. 5 shows a schematic diagram of a third embodiment of a method according to the present invention.
Fig. 6 shows a schematic diagram of a fourth embodiment of a method according to the present invention.
Fig. 7 shows two snapshots of a video of a driver's chest without and with occlusion.
Fig. 8 shows a schematic diagram of a fifth embodiment of a method according to the present invention.
Fig. 9 shows a diagram of different signals used according to the present invention using real data.
Fig. 10 shows diagrams illustrating PCA weights (N x M x 2) visualized on the chest area.
Fig. 11 shows a diagram of different signals used according to the present invention based on synthetic data using spectral subtraction for 2 x 2 blocks.
Fig. 12 shows a diagram of different signals used according to the present invention based on synthetic data using spectral subtraction for 4 x 4 blocks.

DETAILED DESCRIPTION OF EMBODIMENTS

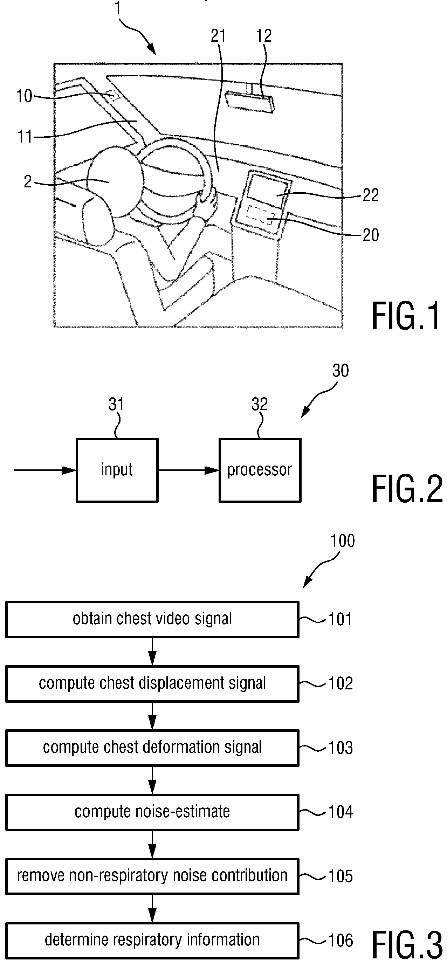

Fig. 1 shows a schematic diagram of an embodiment of a system 1 for determining respiratory information of a subject 2 according to the present invention. In this exemplary embodiment, the system 1 is used for detecting drowsiness or, more generally, the health status or stress status of the driver of a car, for which purpose respiratory information is determined. Respiratory information may preferably be the driver's respiration rate but may additionally or alternatively include one or more of respiration depth, respiration volume, inhalation and exhalation of the driver 2. Based on the determined respiratory information, it can be determined, for instance, if the driver is still fully awake and attentive or is getting tired and drowsy, in which case an alert may be issued, such as an audible and/or visible signal and/or a haptic signal (e.g. vibration of the steering wheel), or it may be recommended to the driver to make a pause.

The system 1 comprises an imaging unit 10 configured to acquire a chest video signal of the subject 2. The imaging unit 10 may, e.g., be a camera that is arranged in the A-pillar 11 (as shown in Fig. 1) or in the rear-view mirror 12 so that it can observe the driver 2 from the front, in particular the driver's chest area. The chest video signal acquired by the imaging unit 10 includes a time series of images of the subject acquired over time, such as a video signal, and covers at least part of the subject's chest area.

The system 1 further comprises a device 20 according to the present invention for determining respiratory information of the subject 2 from the chest video signal. The device 20 may, e.g., be a processor or computer that is, e.g., arranged somewhere within the dashboard 21. The device 20 may, e.g., be combined with the central processing unit of the car entertainment or car navigation system and may use a central display for issuing warnings or recommendations.

Fig. 2 shows a schematic diagram of an embodiment of a device 30 for determining respiratory information of the subject 2 according to the present invention that may be used as device 20 in the system 1 shown in Fig. 1. The device 30 comprises an input 31 configured to obtain a chest video signal of the subject 2 and a processor 32 for processing the obtained chest video signal and determining the respiratory information. The chest video signal may be directly obtained (i.e., received or retrieved) from the imaging unit 10 or from a buffer that temporarily stores the acquired chest video signal or from a preprocessor that preprocesses the chest video signal acquired by the imaging unit 10.

Fig. 3 shows a flowchart of a first embodiment of a method 100 for determining respiratory information of the subject 2 according to the present invention. The steps of the method may be carried out by the device 20 or 30, wherein the main steps of the method are carried out by the processing unit 31. The method 100 may e.g. be implemented as computer program running on a computer or processor. In a first step 101, a chest video signal of the subject is obtained, e.g. directly from the imaging unit 10. In a second step 102, a chest displacement signal is computed from the obtained chest video signal by determining vertical displacements of the chest in a direction substantially along or parallel to the subject's longitudinal body axis. In a third step 103, which may be carried out in parallel, before or after step 102, a chest deformation signal is computed from the obtained chest video signal by determining deformations of the chest. In a fourth step 104, a noise-estimate related to the non-respiratory noise contribution in the chest displacement signal is computed using the chest displacement signal and the chest deformation signal. In a fifth step 105, the non-respiratory noise contribution in the chest displacement signal is - at least partly, preferably as much as possible or even completely - removed by subtraction of the noise-estimate from the chest displacement signal. The obtained noise-reduced (or even noise-free) chest displacement signal can then be used in a sixth step 106 to determine the desired respiratory information and, optionally, to determine the subject's health and/or drowsiness state.

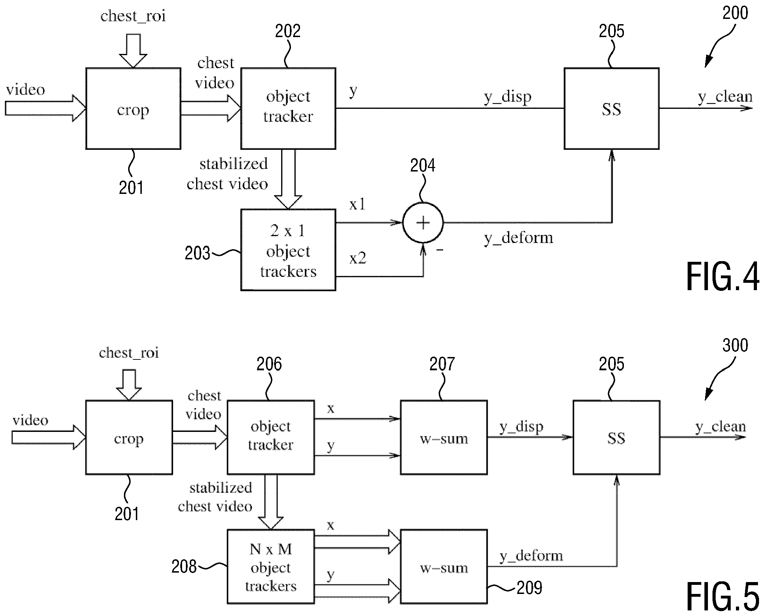

Fig. 4 shows a schematic diagram of a second embodiment of a method 200 for determining respiratory information of the subject 2 according to the present invention. Initially in step 201, the video acquired by the imaging unit may be cropped by use of a chest region-of-interest (ROI) to obtain a video of the chest only, referred to as chest video signal. The chest ROI may be selected a-priori, where it can be assumed that this ROI does not vary over time, since the driver 2 will not change his/her position during driving. Alternatively, the chest ROI may be tracked automatically by e.g. using information of e.g. head-tracking algorithms, shoulder/torso tracking algorithms, seatbelt tracking algorithms, etc.

The chest video signal is processed by one (or more) object tracker(s) in step 202. The object tracker in step 202 operates on the full chest video and aims to stabilize the chest region to get rid of common-mode signals. As a consequence of this step 202, not only the common-mode vibration influences, but also the common-mode respiration influences will be removed from the resulting stabilized chest video. The output signal y from the first object tracker contains the superimposed influences of these common-mode vibrations and the common-mode respiration and represents the chest displacement signal y_disp. Generally, it is a 1D signal.

The stabilized chest video, which generally is a 2D image sequence, is also processed by one (or more) object tracker(s) in step 203. Deformations of the chest are measured using two signals (x1 and x2) produced by tracking two blocks of the chest area in the x-direction (orthogonal to the vertical direction y).

In step 204, the difference of these two signals x1, x2 is computed, the result representing the chest deformation signal y_deform.

The difference signal y_deform is then used in a spectral subtraction (SS) scheme in step 205 to suppress vibrations in the single full-chest signal y_disp in the vertical direction, resulting in a cleaned noise-free (or, at least, noise-reduced) chest displacement signal y_clean.

Fig. 5 shows a schematic diagram of a third embodiment of a method 300 for determining respiratory information of the subject 2 according to the present invention. Steps 201 and 205 of the method 200 are used in the method 300 as well. Steps 202-204 are modified (now referenced as steps 206, 208, 209) and step 207 is added.

In step 206, the object tracker issues two output signals x and y that contain the superimposed influences of these common-mode vibrations and the common-mode respiration.

In order to focus more on the vertical direction of the chest (e.g. in case the camera is not placed perfectly in front of the driver), a weighted summation of the output signals x, y is applied in step 207, where the weights can be both positive or negative values (or even zero). The weights can be found adaptively by using, e.g., a PCA or ICA approach (or any other useful approach to determine the optimal weights. The result is the chest displacement signal y_disp. Another method for finding the optimal weights is to use an analysis of the body posture of the subject to find the longitudinal body axis. Furthermore, the analysis of the body posture of the subject and/or the a-priori known orientation of the camera may be used to correct the sign of the weights to obtain a chest displacement signal where the direction of the output displacement signal is always going upward for inspirations and down for expirations.

In step 208, the stabilized chest video is chopped into a grid of N by M blocks, where each block is fed into a separate object tracker. Hereby, the number of blocks in the x direction is preferably the same as the number of blocks in the y direction, but the number of blocks in these two directions may also be different. Preferably, N x M object trackers are running in parallel to generate N x M signals in the x direction and N x M signals in the y direction.

In step 209, the output signals of step 208 are subjected to a weighted summation, similar as in step 207, where the weights may be found adaptively by using, e.g., a PCA or ICA approach. The result is the chest deformation signal y_deform.

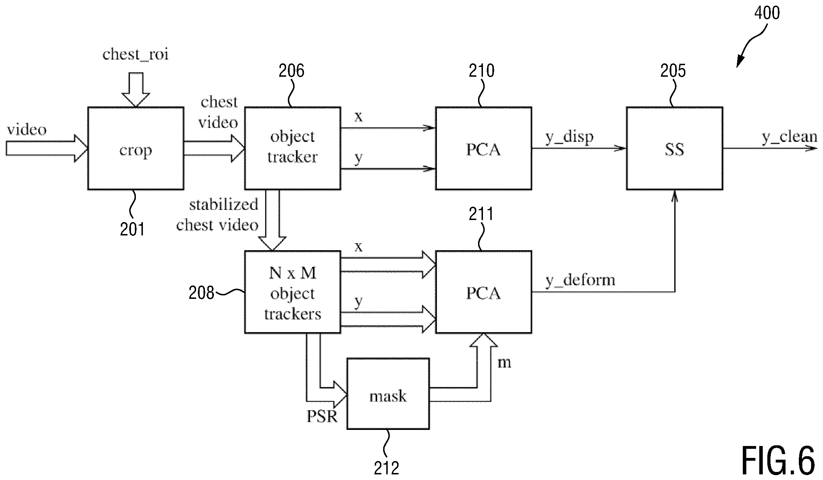

Fig. 6 shows a schematic diagram of a fourth embodiment of a method 400 for determining respiratory information of the subject 2 according to the present invention. Steps 201, 205, 206 and 208 of the method 300 are used in the method 400 as well. Steps 207 and 209 are modified (now referenced as steps 210, 211) and step 212 is added.

In step 210, a PCA technique, as generally known or as e.g. disclosed in E. Oja, "A simplified neuron model as a principal component analyzer", J. Mathematical Biology 1982, vol 15, pp. 267-273, is applied to obtain the strongest signal energy in this 2D space, typically and most strongly in the vertical y-direction of the chest in case of steady driving without posture changes of the driver. A PCA may be seen as a special case of a weighted summation as applied in step 207. The adaptation of this PCA technique does not have to be quick since the weights of the PCA mainly rely on the mounting position of the camera (as pointed out earlier) in combination with a roughly stable position of the passenger or the driver of the car. The weights of the PCA may be constrained such that only a +/- 90 degrees rotation of the x- and y-signal space is allowed, because otherwise the property that the inspirations are always showing upwards as an increase of the respiration signal may be lost.

Before computing the PCA of the signals x and y in step 210, the input signals of step 210 may be ensured to be free of a DC component, for instance by applying (not shown in Fig. 6) a high pass filter with a cut-off frequency that is low enough to let respiration energy pass through. In step 211, a PCA technique is applied as well on the N x M x 2 signals generated by the object trackers in step 208 to obtain the strongest signal energy (other than the common mode that was removed in the stabilization step) that represents the chest deformation signal. Again, a high pass filter may be applied on the input signals of step 211 to remove the DC component. Furthermore, the adaptation of the PCA applied in step 211 can be set quicker than the PCA applied in step 210 since the weights of the PCA can depend on, e.g., lighting conditions that can enhance or deteriorate the contrast of the input to the respective tracker(s) and therefore influence the tracking performance. Furthermore, also the deformation properties of the passenger or the driver of the car can be changing depending on slight positional changes in the seat.

In case of strong occlusions (e.g., hands moving in front of the chest), it may be an option to enable switching off some input signals to the PCA performed in step 211. This is done in this embodiment by a masking vector 'm' that is computed in step 212. Although there are many ways to compute this masking vector, in a preferred embodiment a peak-to-sidelobe ratio (PSR) from the object tracker applied in step 208 may be used, as e.g. disclosed in D.S. Bolme D.S, et.al. "Visual object tracking using adaptive correlation filters", Proc. IEEE Conf. Computer Vision and Pattern Recognition (CVPR), 2010, pp. 2544-2550. In case the PSR of a specific block (out of the N x M blocks used in step 208) drops below a certain (e.g. predefined) threshold, feeding the signals of this particular block to the PCA (including both the x and y signals) applied in step 211 may be switched off. In order to avoid dropping of energy in the output signal of the PCA when switching off one or more blocks, a compensation factor in the output signal of the PCA may be increased to compensate for the loss of energy that would happen otherwise when switching off a certain weight. For this compensation factor it can be assumed that all input signals coherently add up (with or without a negation) to maximize the respiration energy at the output. Another way to deal with missing data (in case specific blocks are switched off due to occlusions) is to use the RPCA (Robust PCA) methods described in the literature, e.g. disclosed in J. Karhunen, "Robust PCA methods for complete and missing data", Neural Network World 5/11, 2010, pp. 357-392.

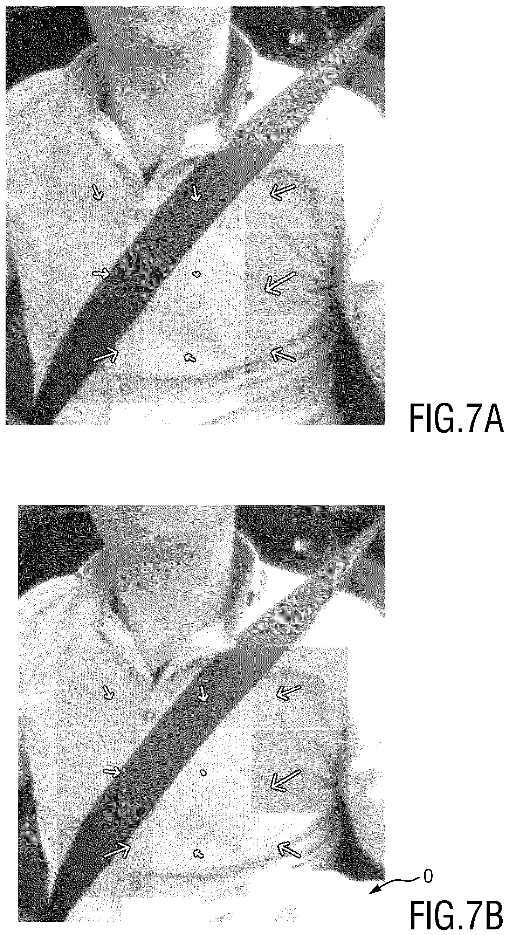

Fig. 7 shows two snapshots of a video of a driver's chest with a visualization of 18 PCA weights in 3 by 3 blocks by means of arrows (one arrow for the x and y weight of a particular block). Fig. 7A shows a typical situation without occlusions, whereas in Fig. 7B there is a visible hand-occlusion O (in the lower right corner) that leads to masking of the most right-lower block (out of the nine blocks) using the masking-vector 'm'.

Via the two PCA steps 210, 211 the chest displacement signal y_disp and the chest deformation signal y_deform are obtained. In the spectral subtraction step 205, these two signals are combined to obtain an estimate of the noise amplitude spectrum $\hat{N}$ from two spectra $Yv$ representing the vertical chest displacement spectrum and $Yd$ representing the chest deformation spectrum that are computed from the output signals of the first and second PCA steps 210, 211, respectively. This noise estimate $\hat{N}$ is then used in a standard spectral subtraction scheme with 50% overlap-and-add, as e.g. disclosed in Boll, S.F, "Suppression of Acoustic Noise in Speech Using Spectral Subtraction", 1979, IEEE Transactions on Acoustic, Speech and Signal Processing, vol 27, pp. 113-120. This noise amplitude spectrum $\hat{N}$ may be subtracted from the input spectrum Yv. The resulting spectrum is then transformed back into a time-domain signal 'y_clean'.

Mathematically, the spectra may be computed as follows:

$$Yv[\kappa] = FFT\{\,\boldsymbol{y}_v\,[\kappa]\,\}, \qquad\qquad (1)$$

and:

$$Yd[\kappa] = FFT\{\,\boldsymbol{y}_d\,[\kappa]\,\}, \qquad\qquad (2)$$

where: $\boldsymbol{y}_v[\kappa]$ and $\boldsymbol{y}_d[\kappa]$ are windowed signal-blocks ($\kappa$ indicating the block-iteration number) constructed from respectively the signals 'y_disp' and 'y_deform' using 50% data-overlapping scheme with previous blocks with iteration $\kappa$ - 1 and using a Hanning window to obtain smoother signal reconstruction in the overlap-and-add output signal.

The noise amplitude spectrum $\hat{N}[\kappa]$ may be computed via:

$$\widehat{N}[\kappa] = max\{\,|Yv[\kappa]| \,-\, \eta\,|Yd[\kappa]|, 0\,\}, \qquad\qquad (3)$$

where $\eta$ is the calibration factor to level the spectral respiration energy of $Yd[\kappa]$ with the spectral respiration energy of $Yv[\kappa]$. For example, the fact may be exploited that in both spectra, the maximum peak in the spectrum corresponds to the majority of the respiration energy. $\eta$ may be computed as:

$$\eta = \frac{|Yv[\kappa, m]|}{|Yd[\kappa, m]|}, \tag{4}$$

where m is the index of the cross-spectrum computed as:

$$\mathrm{m} = \underset{i}{\mathrm{argmax}}\{ \, | \, X_{vd}[\kappa, i] \, | \, \}, \tag{5}$$

where $X_{vd}[\kappa]$ is the cross-spectrum of $Yv[\kappa]$ and $Yd[\kappa]$.

In Eq. (5), the argmax operator may be limited to a range of values of *i*, which are lying inside the spectral region where the majority of the respiratory energy can be expected, e.g. from 0.1 Hz up to 1 Hz.

In the spectral subtraction scheme, a gain-function may be computed as follows:

$$G[\kappa] = \max\left\{ 1 - \frac{\gamma \, \widehat{N}[\kappa]}{|Yv[\kappa]| + \varepsilon}, 0 \right\}, \tag{6}$$

where $\gamma$ is an over-subtraction factor, wherein $\gamma \geq 1$ and $\varepsilon > 0$ is a small value to prevent division by zero. It can be seen that the gain function will be close or equal to zero in case the spectral energy of the noise estimate is close or larger than the spectrum $|Yv[\kappa]|$.

The gain-function may be applied to the spectrum $Yv[\kappa]$ by:

$$Y[\kappa] = G[\kappa] \bullet Yv[\kappa], \tag{7}$$

where • is the element-wise multiplication operator.

The final output-signal may be constructed as:

$$y_{clean}[\kappa] = IFFT\{Y[\kappa]\}, \tag{8}$$

where the most recent half of time-samples are stored for the next iteration $\kappa + 1$ and the oldest half of time-samples are combined with the stored time-samples of the previous iteration $\kappa - 1$ to construct the output signal 'y_clean'.

The spectral subtraction may also be applied in the generalized power domain having arbitrary exponents in the magnitude |X|, i.e. |X|^alpha, where alpha can have any positive non-integer values, as, e.g., described in M. Berouti, "Enhancement of speech corrupted by acoustic noise," in Proceedings of the IEEE International Conference on Acoustics, Speech, and Signal Processing (ICASSP '79), pp. 208-211, 1979.

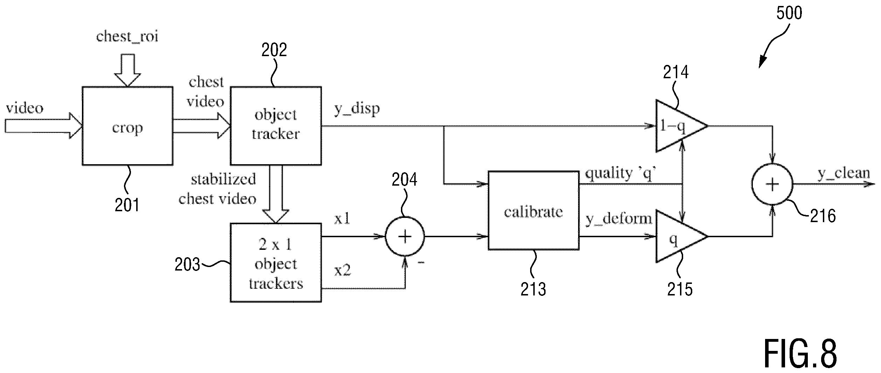

Fig. 8 shows a schematic diagram of a fifth embodiment of a method 500 for determining respiratory information of the subject 2 according to the present invention. Steps 201 to 204 of the method 200 are used in the method 500 as well. Steps 213 to 216 are added. According to this embodiment, the computation of y_clean is, as an alternative to the spectral subtraction, be done in the time-domain via a weighted combination (step 216) of y_disp and y_deform where y_deform is calibrated in step 213 to match the phase and magnitude of the respiratory contribution in the chest deformation signal and the chest displacement signal. From the finally obtained signal y_clean, the desired respiratory information may then be computed.

The quality of the calibration indicated by q sets the two weighting values of the weighting of y_disp and y_deform to respectively 1-q and q in steps 214 and 215. In case of a poor calibration where y_deform does not contain any respiratory contribution matching with the respiratory contribution of y_deform, q=0 is set resulting in y_clean = y_disp. In case of a good calibration where the respiratory contribution in y_deform nicely correlates with the respiratory contribution in y_disp, q=1 is set resulting in y_clean = y_deform. Intermediate values of q will lead to a mixture of y_clean and y_deform.

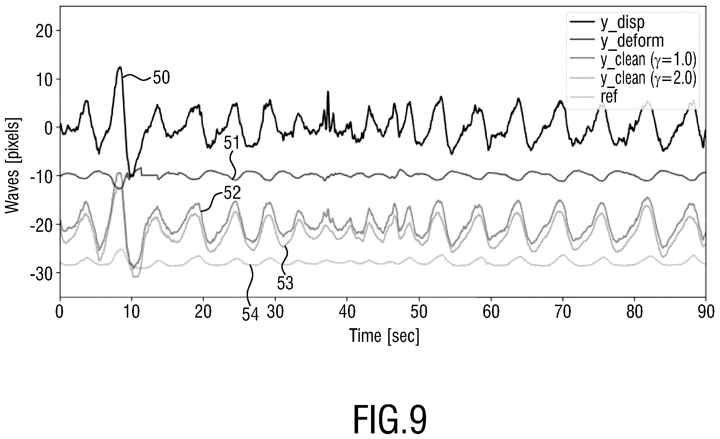

Fig. 9 shows a diagram of different signals used according to the present invention using a 90 second snippet recorded in a moving car to illustrate the noise reduction using spectral subtraction. Curve 50 shows the original displacement signal 'y_disp'. As can be seen, there is quite some noise in the signal, because the car is moving on the highway. Curve 51 shows the deformation signal 'y_deform' that is obtained via the PCA with 3 by 3 blocks, which is used in the spectral subtraction to obtain the noise estimate via Eq. (3). As can be seen from this deformation signal 51, the phase has been flipped (180 degrees phase difference) with respect to the displacement signal 50. Curves 52 and 53 show the result 'y_clean' after spectral subtraction using different over-subtraction factors $\gamma$. Curve 52 has a marginal subtraction using $\gamma$ = 1.0, whereas curve 53 has an over-subtraction using $\gamma$ = 2.0. As can be seen, the phase of the cleaned waves matches with the original displacement signal 50 due to the fact that the spectral subtraction only modifies the amplitude spectrum of the original displacement signal 50. Curve 54 shows the reference signal that was obtained by a respiband signal and has arbitrary units (not pixels).

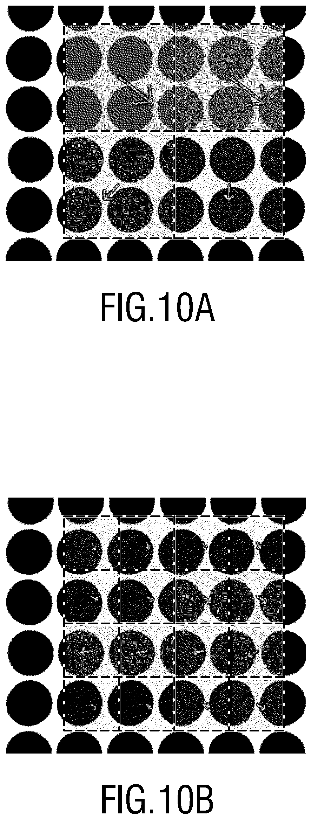

Although the real data example shown in Fig. 9 (recorded on the highway) shows the effect of the spectral subtraction in practice, displacements with noise and deformations with noise can be simulated to mimic a more severe/noisy situation. As synthetic data, a video (at 15 fps) has been generated having a dynamic circle-board pattern having vertical motion and deformations, as shown in Fig. 10 illustrating PCA weights (N x M x 2) visualized on the chest-area. These motions and deformations are modulated using a simulated respiration wave of 0.25 Hz. 5 pixels of vertical pixel motion and only 2 pixels of chest deformation are applied, which is rather similar to the real example illustrated in Fig. 9. Furthermore, a vertical vibration displacement of 10 pixels is used, modelled as random noise which is to be removed by the spectral subtraction.

The chest deformations are modelled as horizontal pixel deformations that deform as a sinusoidal pattern as function of the vertical axis with a peak-to-peak pixel deformation of 4 pixels. The sinusoidal pattern of deformations spans roughly 1.5 periods over the vertical axis of the chest area selection. As a result of this granularity, the PCA weights are better conditioned in case of more blocks, as can be seen in Fig. 10, where the 4 x 4 blocks case illustrated in Fig. 10B better captures the chest deformation compared to the 2 x 2 blocks case illustrated in Fig. 10A.

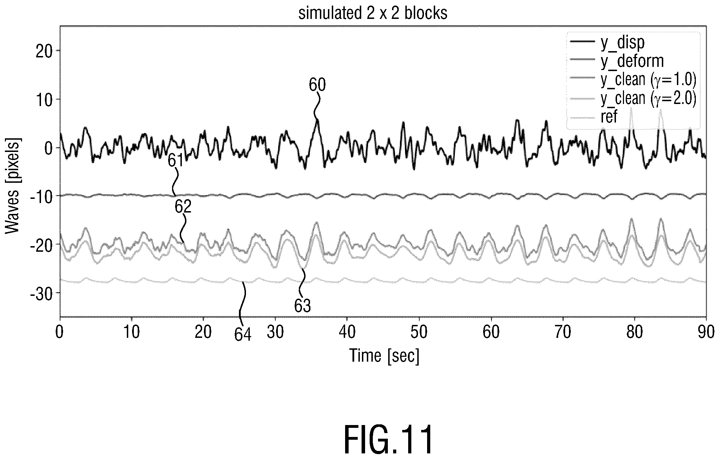

Fig. 11 shows a diagram of different signals used according to the present invention using synthetic data to illustrate the noise reduction using spectral subtraction for 2 x 2 blocks (N=M=2). By using the synthetic data, the same algorithm is applied as for the real data example illustrated in Fig. 9. Similar to Fig. 9, Fig. 11 shows a displacement signal 60 and a deformation signal 61 (both obtained by PCA). The convergence of the PCA weights can be seen in the initial part of the deformation signal 61. The spectral subtraction results are again shown in the curve 62 and 63, where curve 62 indicates the result of using $\gamma = 1.0$, whereas curve 63 indicates the result of using $\gamma = 2.0$. The (artificially generated) reference signal 64 is shown at the bottom having arbitrary units.

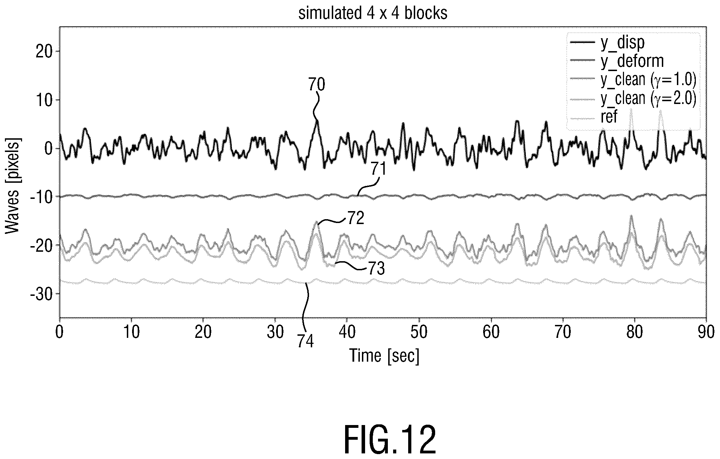

Fig. 12 shows a diagram of different signals used according to the present invention using synthetic data to illustrate the noise reduction using spectral subtraction for 4 x 4 blocks (N=M=4). This diagram shows that it is beneficial to choose the number of blocks in the PCA according to the granularity of the chest deformations. Similar to Fig. 11, Fig. 12 shows the following signals: displacement signal 70, deformation signal 71, clean signal 72 using $\gamma = 1.0$, clean signal 73 using $\gamma = 2.0$, reference signal 74.

In summary, the present invention allows a more robust and accurate determination of respiratory information. The present invention assesses chest deformations in the chest region for use in the determination of the respiratory information. Since the deformation signal typically has a poorer signal to noise ratio compared to the chest displacement signal and also because there is an inherent sign ambiguity (inspiration versus expiration), the two signals are combined. A noise estimate of the vibrations in the displacement signal is computed. Subsequently, this estimated noise is subtracted from the displacement signal, preferably by using spectral subtraction.

The invention can be used in micro-motion vital sign measurements in various applications, including ambulatory applications and automotive applications to monitor the condition of the driver.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A method for determining respiratory information of a subject, the method comprising:

    - obtaining a chest video signal of the subject, the chest video signal including a time series of images of the subject acquired over time and covering at least part of the subject's chest area;
    - computing a chest displacement signal from the obtained chest video signal by determining vertical displacements of the chest in a direction substantially along or parallel to the subject's longitudinal body axis;

- computing a chest deformation signal from the obtained chest video signal by determining deformations of the chest;
- computing a noise-estimate related to the non-respiratory noise contribution in the chest displacement signal using the chest displacement signal and the chest deformation signal;
- removing at least part of the non-respiratory noise contribution in the chest displacement signal by subtraction of the noise-estimate from the chest displacement signal to obtain a noise-reduced chest displacement signal; and
- determining the respiratory information from the noise-reduced chest displacement signal.

2. The method according to claim 1,
wherein the chest displacement signal is computed using an object tracker on the chest area to generate a vertical tracking signal corresponding to the displacements of the chest in the vertical direction.

3. The method according to claim 1 or 2,
wherein the step of computing the chest deformation signal includes:

- using at least two object trackers on the chest, each object tracker tracking a different part of the chest area in two dimensions to generate at least two tracking signals in orthogonal directions;
- removing common-mode displacements in the at least two tracking signals; and
- combining the at least two tracking signals via a weighted summation.

4. The method according to any one of the preceding claims,
wherein the steps of computing the noise-estimate and removing the non-respiratory noise contribution are performed in the time domain and include:

- computing a calibrated chest deformation signal from the chest deformation signal and the chest displacement signal to match the phase and magnitude of the respiratory contribution in the chest deformation signal and the chest displacement signal;
- estimating the noise signal from the calibrated chest deformation signal and the chest displacement signal;
- computing a weighted noise-estimate signal, where the weight varies from 0 to 1 and the weight is representing the similarity of the respiratory contribution in the calibrated chest deformation signal and the chest displacement signal; and
- removing the non-respiratory noise contribution in the chest displacement signal by subtraction of the weighted noise-estimate from the chest displacement signal.

5. The method according to any one of claims 1 to 3,
wherein the steps of computing the noise-estimate and removing the non-respiratory noise contribution are performed in the spectral domain and include:

- computing the chest displacement spectrum from the chest displacement signal;
- computing the chest deformation spectrum from the chest deformation signal;
- estimating a noise spectrum related to the non-respiratory noise contribution in the chest displacement spectrum using the chest displacement spectrum and the chest deformation spectrum; and
- modifying the spectral energy in the chest displacement spectrum using the estimated noise spectrum.

6. The method according to claim 5,
wherein modifying the spectral energy in the chest displacement spectrum includes:

- computing the chest displacement magnitude spectrum; and
- subtracting the noise spectrum from the chest displacement magnitude spectrum using spectral subtraction.

7. The method according to claim 6,
wherein the spectral subtraction uses an over-estimation of the noise spectrum with a value larger than 1.

8. The method according to any one of claims 5 to 7,
wherein estimating the noise spectrum related to the non-respiratory noise contribution in the chest displacement spectrum includes:

- computing a calibrated chest deformation spectrum from the chest deformation spectrum and the chest displacement spectrum; and

- estimating the noise spectrum from the calibrated chest deformation spectrum and the chest displacement spectrum.

9. The method according to claim 8,

   wherein the calibrated chest deformation spectrum is computed by multiplying the chest deformation spectrum with a calibration factor; and
   wherein the noise spectrum is computed by subtracting the calibrated chest deformation magnitude spectrum from the chest displacement magnitude spectrum and clipping the negative values of the noise spectrum to zero.

10. The method according to claim 9,
    wherein the calibration factor is computed by

    - identifying the harmonic frequency that has the strongest joint energy contribution in both the chest displacement spectrum and the chest deformation spectrum; and
    - computing the calibration factor by dividing the magnitude of the identified harmonic frequency in the chest displacement spectrum by the magnitude of the identified harmonic frequency in the chest deformation spectrum.

11. The method according to any one of the preceding claims,
    further comprising selecting and/or tracking the chest area in the time series of images of the subject to generate the chest video signal.

12. The method according to any one of the preceding claims,
    wherein the respiratory information comprises one or more of respiration rate, respiration depth, respiration volume, inhalation and exhalation of the subject.

13. A device for determining respiratory information of a subject, the device comprising:

    an input configured to obtain a chest video signal of the subject, the chest video signal including a time series of images of the subject acquired over time and covering at least part of the subject's chest area; and
    a processor configured to

       - compute a chest displacement signal from the obtained chest video signal by determining vertical displacements of the chest in a direction substantially along or parallel to the subject's longitudinal body axis;
       - compute a chest deformation signal from the obtained chest video signal by determining deformations of the chest;
       - compute a noise-estimate related to the non-respiratory noise contribution in the chest displacement signal using the chest displacement signal and the chest deformation signal;
       - remove at least part of the non-respiratory noise contribution in the chest displacement signal by subtraction of the noise-estimate from the chest displacement signal to obtain a noise-reduced chest displacement signal; and
       - determine the respiratory information from the noise-reduced chest displacement signal.

14. A system for determining respiratory information of a subject, the system comprising:

    - an imaging unit configured to acquire a chest video signal of the subject, the chest video signal including a time series of images of the subject acquired over time and covering at least part of the subject's chest area; and
    - a device according to claim 13 for determining respiratory information of the subject from the chest video signal.

15. A computer program comprising program code means for causing a computer to carry out the steps of the method as claimed in any one of claims 1 to 12 when said computer program is carried out on the computer.

FIG.1

FIG.2

| obtain chest video signal | 101 |
| compute chest displacement signal | 102 |
| compute chest deformation signal | 103 |
| compute noise-estimate | 104 |
| remove non-respiratory noise contribution | 105 |
| determine respiratory information | 106 |

FIG.3

FIG.4

FIG.5

EP 4 678 099 A1

FIG.6

FIG.7A

FIG.7B

FIG.8

FIG.9

EP 4 678 099 A1

FIG.10A

FIG.10B

FIG.11

EP 4 678 099 A1

EP 4 678 099 A1

FIG.12

EP 4 678 099 A1

Europäisches Patentamt
European Patent Office
Office européen des brevets

# EUROPEAN SEARCH REPORT

**Application Number**

EP 24 18 7640

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WARNECKE JOANA M ET AL: "Noise Reduction for Efficient In-Vehicle Respiration Monitoring with Accelerometers", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23), pages 3257-3261, XP033625560, DOI: 10.1109/EMBC.2019.8857654 [retrieved on 2019-10-02] * Title; Abstract; Sections: "B. Data Processing", "IV. DISCUSSION"; figure 1 * | 1-15 | INV. A61B5/08 A61B5/11 A61B5/113 A61B5/00 |
| A | WANG WENJIN ET AL: "Algorithmic insights of camera-based respiratory motion extraction", 20220707, vol. 43, no. 7, 7 July 2022 (2022-07-07), XP020462664, DOI: 10.1088/1361-6579/AC5B49 [retrieved on 2022-07-07] * section: "4.Motion extraction strategies"; figure 4 * | 1-15 | |
| X | CN 117 462 111 A (UNIV HEFEI TECHNOLOGY) 30 January 2024 (2024-01-30) * paragraphs [0038], [0048] - [0057] * | 1-15 | |

TECHNICAL FIELDS
SEARCHED (IPC)

A61B

The present search report has been drawn up for all claims

| Place of search | Date of completion of the search | Examiner |
|---|---|---|
| The Hague | 8 November 2024 | Papanikolaou, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 7640

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2024

| Patent document cited in search report | Publication date | Patent family member(s) | Publication date |
|---|---|---|---|
| CN 117462111 A | 30-01-2024 | NONE | |

EPO FORM P0459

For more details about this annex : see Official Journal of the European Patent Office, No. 12/82

**REFERENCES CITED IN THE DESCRIPTION**

**Non-patent literature cited in the description**

- **M. VILLARROEL**. Non-contact vital-sign monitoring of patients undergoing haemodialysis treatment. *Sci rep.*, 2020, vol. 10, 18529, https://doi.org/10.1038/s41598-020-75152-z
- **H. HWANG**. A Real-Time Remote Respiration Measurement Method with Improved Robustness Based on a CNN Model. *Appl. Sci.*, 2022, vol. 12 (11603), 1-27
- **S. N. GRAMA** ; **S. J. SUBRAMANIAN**. *Computation of Full-field Strains Using Principal Component Analysis*, July 2014
- **S.F. BOLL, S.F**. Suppression of Acoustic Noise in Speech Using Spectral Subtraction. *IEEE Transactions on Acoustic, Speech and Signal Processing*, 1979, vol. 27, 113-120
- **E. OJA**. A simplified neuron model as a principal component analyzer. *J. Mathematical Biology*, 1982, vol. 15, 267-273
- **BOLME D.S**. Visual object tracking using adaptive correlation filters. *Proc. IEEE Conf. Computer Vision and Pattern Recognition (CVPR)*, 2010, 2544-2550
- **J. KARHUNEN**. Robust PCA methods for complete and missing data. *Neural Network World*, 2010, vol. 5 (11), 357-392
- **BOLL, S.F**. Suppression of Acoustic Noise in Speech Using Spectral Subtraction. *IEEE Transactions on Acoustic, Speech and Signal Processing*, 1979, vol. 27, 113-120
- **M. BEROUTI**. Enhancement of speech corrupted by acoustic noise. *Proceedings of the IEEE International Conference on Acoustics, Speech, and Signal Processing (ICASSP '79)*, 1979, 208-211